# EUROPEAN PATENT SPECIFICATION

(11) **EP 3 920 892 B1**
(45) Date of publication and mention of the grant of the patent: **15.11.2023**
(21) Application number: 20752394.5
(22) Date of filing: 04.02.2020
(51) Int. Cl.: A61K 9/20, A61K 31/315, A61K 31/7076, A61K 47/26, A61K 47/36, A61P 31/12, A61P 37/04, A61K 31/133, A61K 31/708, A61K 33/30, A61K 31/196

(54) **A SOLID DOSAGE FORM COMPRISING ZINC GLUCONATE AND INOSINE PRANOBEX, A METHOD FOR ITS PREPARATION AND ITS APPLICATIONS**
FESTE DOSIERUNGSFORM MIT ZINKGLUCONAT UND INOSIN-PRANOBEX, VERFAHREN ZU IHRER HERSTELLUNG UND IHRE ANWENDUNGEN
FORME POSOLOGIQUE SOLIDE COMPRENANT DU GLUCONATE DE ZINC ET DE L'INOSINE PRANOBEX, ET PROCÉDÉ DE PRÉPARATION ET SES APPLICATIONS

(30) Priority: 04.02.2019 PL 42879819
(43) Date of publication of application: 15.12.2021
(73) Proprietor: Aflofarm Farmacja Polska SP. Z O.O., 95-200 Pabianice (PL)
(72) Inventor: WAHL, Hanna, 95-200 Pabianice (PL); DABROWA, Marek, 90-146 Lódz (PL); LASKIEWICZ-RURAZ , Monika, 98-100 Lask (PL); DANIELSKA, Paulina, 94-058 Lódz (PL); GABLASINSKA, Justyna, 93-247 Lódz (PL); MADEJCZYK, Arkadiusz, 95-054 Ksawerów (PL); KULAZINSKI, Piotr, 95-200 Pabianice (PL); PASINSKI, Jarek, 90-625 Lódz (PL)
(74) Representative: Witek, Rafal
(86) International application number: PCT/PL2020/050014
(87) International publication number: WO 2020/162773

(56) References cited:
- WO-A1-2019/013658
- WO-A1-2019/013658
- CN-B- 103 120 648
- JP-A- 2015 063 521
- Anonymous: "Charakterystyka produktu leczniczego", polfarmex , 1 August 2016 (2016-08-01), pages 1-6, XP055838925, Retrieved from the Internet: URL:https://www.polfarmex.pl/zasoby/produk ty_ulotki_chpl/eloprine_tabl.500mg_chpl.pd f

## Description

The present invention relates to a solid dosage form comprising zinc gluconate and inosine pranobex, a method for its preparation and its applications. The invention is applicable in pharmacy.

In the state of the art, there is a known drug called Eloprine in the form of a tablet, manufactured by Polfarmex SA, containing in addition to inosine pranobex, also mannitol, potato starch, povidone K-25 and magnesium stearate. There is a known drug called Groprinosin from Gedeon Richter Polska Sp z o.o. in the form of a tablet containing the same active substance and excipients, such as potato starch, povidone K-25 and magnesium stearate. Another drug in the same form containing the same active substance is Neosine from Aflofarm Farmacja Polska Sp z o.o., which additionally contains wheat starch, mannitol, povidone and magnesium stearate as excipients. A therapeutic composition is known from the international application WO2012096655A1, comprising alkylglycerols, atremidine, *Agaricus bisporus,* chlorophylline, inosine and zinc. For a medicine to have a therapeutic effect on humans, the active substances it contains must be released from the medicine (e.g. tablets) in which they are administered to the patient, then they must be dissolved in an acceptor fluid (e.g. stomach content) so that they can be absorbed into the blood later, with the help of which they will reach the appropriate receptor causing a specific clinical effect. It follows that the time of releasing the active substance from the developed drug form and the amount of the substance which will be released in a given time is extremely important, therefore, at the stage of developing new drug forms, special attention should be paid to the kinetics of release of active substances. There are methods to measure the concentration profile of the substance released from the time it takes place using laboratory methods. At the beginning of the development of a new form of the drug containing inosine pranobex and zinc gluconate, it was assumed that the clinical effect of inosine pranobex should be the same as in products where it is the only active substance, therefore the formulation task was to develop a new form from which the inosine pranobex release profile will be consistent with the release profile of this substance in market monoproducts and zinc ions will be released from the same form of the drug in at least 80% of the set amount in 45 min. The combination of inosine pranobex and zinc gluconate in one product is clinically relevant as the effect of inosine pranobex in the event of zinc deficiency may be reduced. Adequate zinc concentration conditions the correct immune response, while inosine pranobex will additionally strengthen it. Patent WO 2019/013658 A1 discloses zinc and inosine pranobex in liquid form. It CN 103 120 648 B discloses a monopreparation of inosine pranobex under the form of a solid tablet.

Preliminary pre-formulation studies have shown that the addition of zinc gluconate to existing formulations of solid drug forms containing inosine pranobex caused a significant slowdown in the release profile of the active substance from the formulation. This translates into a deterioration of the pharmacokinetic parameters of the product, which may result in a worse therapeutic effect. There is still a need to provide a pharmaceutical composition with inosine pranobex and zinc gluconate and its method of preparation, which conditions the proper functioning of the immune system, while its solid form has appropriate release profiles of inosine pranobex (consistent with the reference product) and a final release of zinc ions of at least 80 % of the set amount in 45 min. Surprisingly, it turned out that the solution to the above problem: too slow release of inosine pranobex from a solid oral drug formulation, containing a combination of two active substances of inosine pranobex and zinc gluconate, compared to the inosine pranobex monopreparations available on the market, proved to be a pharmaceutical composition and a method of its preparation presented in this description.

The first subject of the invention is a solid dosage form containing zinc gluconate and inosine pranobex and adjuvants, characterized in that zinc gluconate is in an amount of 3.11% to 4.51% by mass of the dosage form, inosine pranobex is in an amount of 71.43% by mass of the dosage form, sodium carboxymethyl starch (type A) is in the range of 5% to 14% by weight of the dosage form, sodium lauryl sulfate is in an amount of 0.7% to 1% by weight of the dosage form, and the mannitol content is from 6% to 10.7% by weight of the dosage form. Everywhere in the patent, mass percentages are used, expressed as the mass of the solid dosage form. Equally preferably, the form according to the invention is characterized in that it contains magnesium stearate, preferably in an amount of 1%, povidone, preferably in an amount of 3.43% and preferably wheat starch in an amount of 3.60% .

A second object of the invention is a method for preparing a solid dosage form, as defined in the first object of the invention, characterized in that it comprises:
a) forming a gruel for the wet granulation process, preferably in an amount of 6.06% by weight of the dosage form based on water, povidone 30 and sodium lauryl sulfate, preferably at povidone temperature from 30 °C to 60 °C, lauryl sulfate temperature from 30 °C to 35 °C, for 3.5 hours
b) pre-mixing of inosine pranobex with zinc gluconate, wheat starch and sodium carboxymethyl starch (type A), preferably at a temperature of 20 °C to 30 °C and with granulator rotating at 100 to 300 rpm
c) spraying the gruel based binder from step a), preferably in the amount determined by the following parameters: pump rotation from 125 rpm to 250 rpm and addition of the binder to the substances from step b) for 2.10 min to 4.40 min, to make granulate
d) drying the granulate from step c), preferably by means of a fluid bed dryer at from 40°C to 50°C
e) mixing the granulate from step d) with mannitol in dry form, preferably sieved through a 1.5 mm to 2.5 mm mesh
f) mixing the granulate from step e) with a lubricant, preferably magnesium stearate, preferably sieved through a 1.5 mm to 2.5 mm mesh
g) tableting, preferably at 10-50 kN pressure forces The third object of the invention is the dosage form as defined in the first object of the invention for use as a medicament intended to modulate the immune response and antiviral activity. The dosage form is administered to a human in amounts that ensure the intake of 500 mg to 1000 mg of inosine pranobex in one solid drug form and from 21.78 mg to 63.16 mg of zinc gluconate in one solid dosage form. Most preferably, the solid dosage from according to the invention is characterized in that the recommended dose is 50 mg per kg of body weight per day of inosine pranobex administered with 1 tablet containing 1000 mg of inosine pranobex and 6.25 mg of zinc ions or 2 tablets containing 500 mg of inosine pranobex and 3.125 mg of zinc ions, 3 to 4 times per day in the adult population, and a half tablet containing 1000 mg inosine pranobex and 6.25 mg zinc ions or 1 or half tablet containing 500 mg inosine pranobex and 3.125 mg zinc ions, 3 to 4 times per day in pediatric population.

### Example 1. Method for obtaining a solid oral drug formulation containing a combination of inosine pranobex and zinc gluconate.

The subject of the invention is a product containing a combination of inosine pranobex and zinc gluconate, characterized by a consistent release profile in relation to monoproducts containing inosine pranobex and final release of zinc ions at the appropriate level, i.e. min 80% within 45 min. The pharmaceutical composition of the medicinal product in solid oral dosage form is presented in Table 1:

**Table 1. Composition of Inosine pranobex + Zinc gluconate, 1000 mg + 43.56 mg tablet.**

| **Lp.** | **Starting materials** | **Functions** | **Amount [mg/tablet]** | | **Amount [%/tablet]** |
|---|---|---|---|---|---|
| 1. | Inosine pranobex | Active substance | 1000.00 | 500.00 | 71.43 |
| 2. | Zinc gluconate | Active substance | 43.56 | 21.78 | 3.11 |
| 3. | Wheat starch | Filling agent | 50.44 | 25.22 | 3.6 |
| 4. | Povidone 30 | Binding agent | 48.00 | 24.00 | 3.43 |
| 5. | Sodium carboxymethyl starch (Type A) | Disintegrating agent | 98.00 | 49.00 | 7.00 |
| 6. | Sodium lauryl sulfate | Excipient that increases the rate of API dissolution | 14.00 | 7.00 | 1.00 |
| 7. | Mannitol | Filling agent | 132.00 | 66.00 | 9.43 |
| 8. | Magnesium stearate | Gliding agent | 14.00 | 7.00 | 1.00 |

The method of preparing a solid oral pharmaceutical composition containing a combination of two active ingredients of inosine pranobex and zinc gluconate is as follows. The preparation process begins with weighing the listed starting materials included in the tested product, according to Table 1. The present invention is prepared based on the wet granulation process, where the granulation solution is an aqueous solution of Povidone 30 and sodium lauryl sulfate ("gruel"). Some raw materials (inosine pranobex; zinc gluconate; wheat starch; sodium carboxymethyl starch (Type A)) are added into the granulator bowl and subjected to preliminary mixing. In the next wet granulation step, the starting materials are sprayed with a binder solution composed of: povidone 30, sodium lauryl sulfate and purified water. The end of this step is the production of granulate. The obtained granulate is dried in a fluid bed dryer. After unloading the drying chamber, the finished granulate is transferred to a mixing container. Mannitol in dry form is added by sieving into the container with granules. The next production step is the mixing step. Magnesium stearate is added to the container with the aforementioned mixture and the whole mixture is mixed again. The tableting mix prepared in this way is tableted using a rotary tablet press. The manufacturing process completes the blister stage of loose product in a direct blister pack, followed by unit packaging in cardboard boxes.

### Example 2. Dissolution testing of a solid oral drug formulation containing a combination of inosine pranobex and zinc gluconate vs monopreparation with inosine pranobex.

The initial composition of the product was developed based on the compositions of market products containing inosine pranobox. At the beginning of developmental studies, the consistency of the release profile of the active substance pranobex inosine in Inosine pranobex + Zinc gluconate, 1000 mg + 6.25 mg and tablets with the reference product was examined. The results of the release profiles of the active substance inosine pranobex are presented in Table 2.

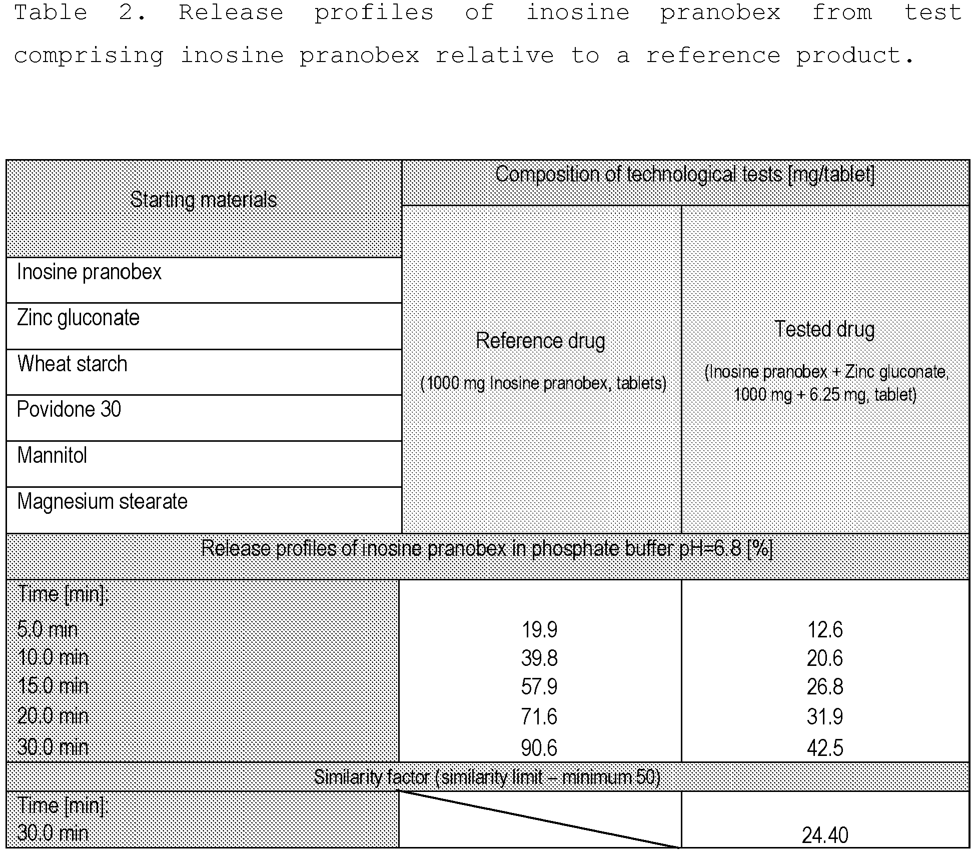

In the technological test of Inosine pranobex + Zinc gluconate product, 1000 mg + 6.25 mg tablet, based on the composition of excipients of the reference product, tablets were obtained which were characterized by a lack of consistency of the inosine pranobex release profile with the active substance release profile of the reference product. Release of API from the tested product is too slow, similarity coefficient is below the similarity limit of minimum 50, which prevents the use of this form of the drug as a therapeutically effective market solution.

### Example 3. Development of a solid oral drug formulation containing a combination of inosine pranobex and zinc gluconate, and substances that accelerate the release of active substances from the solid drug form.

Since it was surprisingly found that the addition of a small amount, relative to the weight of the whole tablet, of zinc gluconate to the inosine pranobex product reduced the pharmaceutical availability of the second active substance, the next stage of the development of the medicinal product was to develop a formulation using excipients with disintegrating properties, i.e. sodium carboxymethyl starch type A, croscarmellose sodium, Kollidon CL, sodium hydrogen phosphate and/or increasing the dissolution rate of API, such as: sodium lauryl sulfate, Tween 80, Brij 58, Poloxamer 188 and PEG 40.

### Example 3a. Use of disintegrating agents (sodium carboxymethyl starch (type A)).

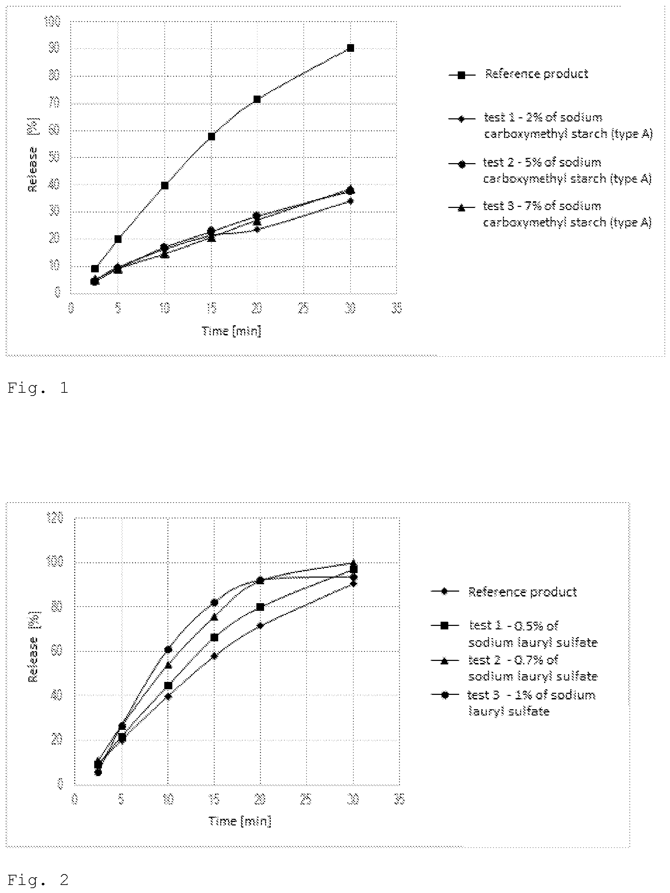

In order to select the amount of the disintegrating agent sodium carboxymethyl starch (type A), technological tests were carried out using a different percentage of this substance in the quantitative composition of the product tested. The composition of tests and the results of the release profiles of the active substance pranobex inosine are shown in Table 3 and Figure 1, where the results of testing the inosine pranobex release profiles in tests with different percentages of sodium carboxymethyl starch (type A) used, in phosphate buffer pH = 6.8, are shown.

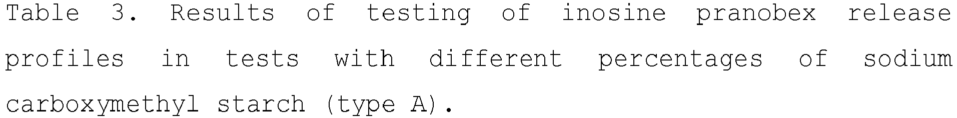

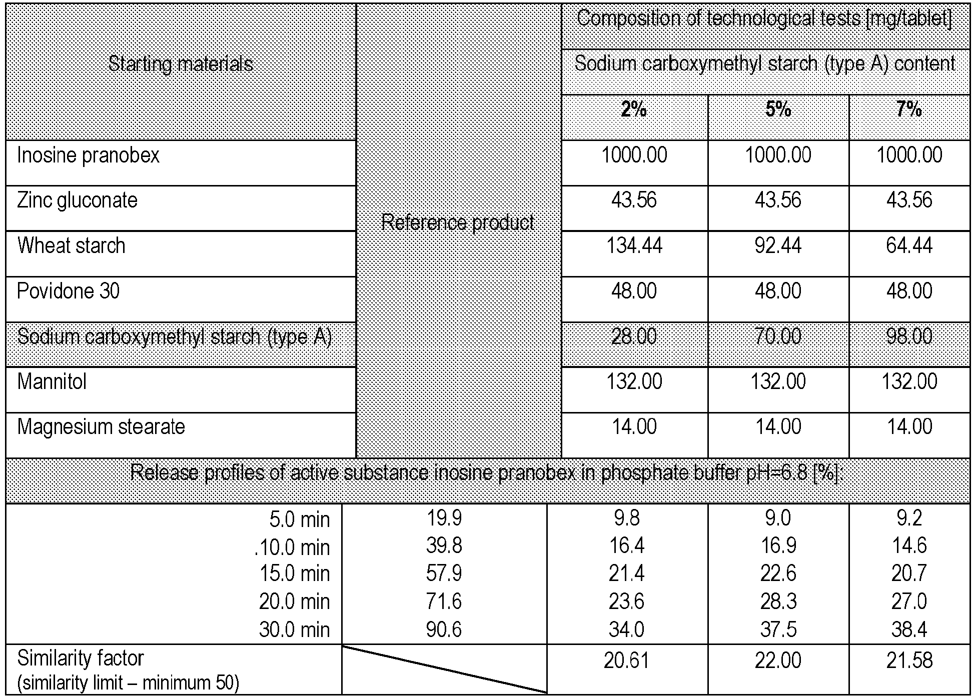

The above results of studies on the release profile of inosine pranobex in a medicinal product containing a combination of two active ingredients: inosine pranobex (1000 mg) and zinc ions (6.25 mg) in the form of zinc gluconate, in the form of a tablet, showed inconsistency of the release profile of the active substance inosine pranobex with reference product.

It was concluded that the use of only sodium carboxymethyl starch (type A) as a disintegrating agent in the final product Inosine pranobex + Zinc gluconate, 1000 mg + 6.25 mg tablet, does not match the release profile of the active substance inosine pranobex, which is a critical parameter proving the therapeutic effectiveness of the studied drug. Surprisingly, it turned out that in this case the active substance inosine pranobex releases more slowly compared to its release from the reference product as well as from the tested product which did not contain sodium starch glycolate (type A).

### Example 3b. Use of a substance that increases the dissolution rate of the active substances (sodium lauryl sulfate)

In order to select the amount of sodium lauryl sulfate, the substance increasing the dissolution rate of the active substances, technological tests were carried out using different percentages of this substance in the quantitative composition of the tested product. The composition of the tests and the results of the release profiles of the active substance pranobex inosine are shown in Table 4 and Figure 2, showing the results of testing the inosine pranobex release profiles in tests with different percentages of sodium lauryl sulfate in phosphate buffer pH = 6.8.

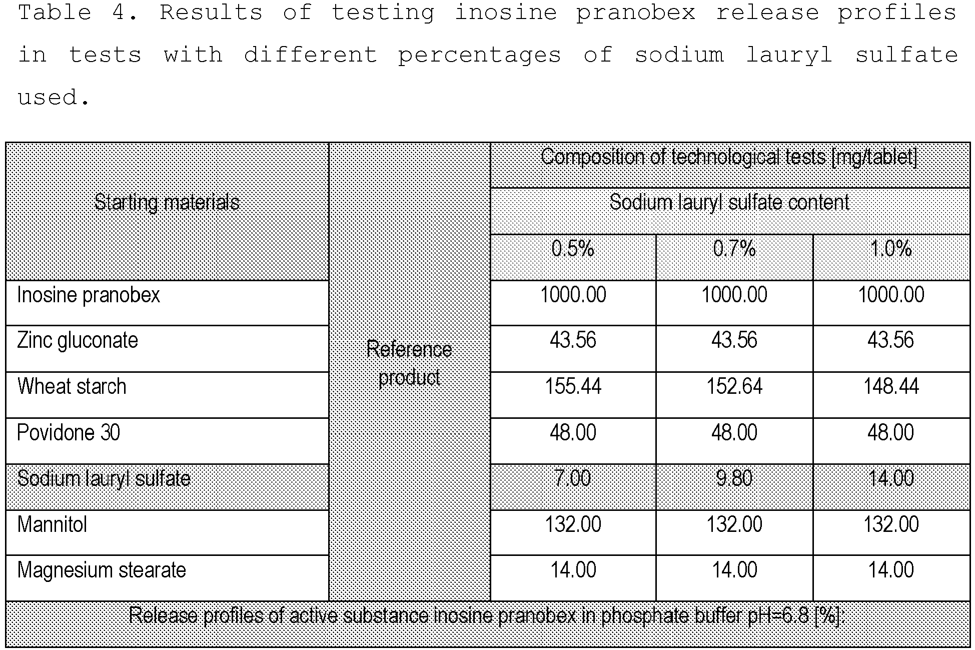

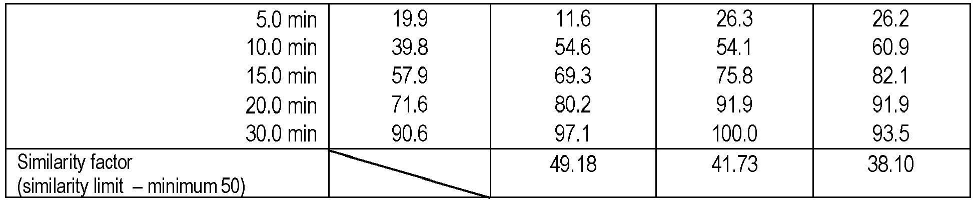

The above results of studies on the release profile of inosine pranobex in a medicinal product containing a combination of two active substances: inosine pranobex (1000 mg) and zinc ions (6.25 mg) in the form of zinc gluconate, in solid form of the drug, showed inconsistency of the release profile of the active substance pranobex inosine with a reference product.

The use of only sodium lauryl sulfate in the final product does not match the release profile of the active substance inosine pranobex. In the conducted tests, the active substance inosine pranobex releases faster compared to its release in the reference product.

### Example 3c. Determination of the appropriate ratio of disintegrants and excipients increasing the dissolution rate of API (sodium carboxymethyl starch (type A) and sodium lauryl sulfate) in the formulation of the test product.

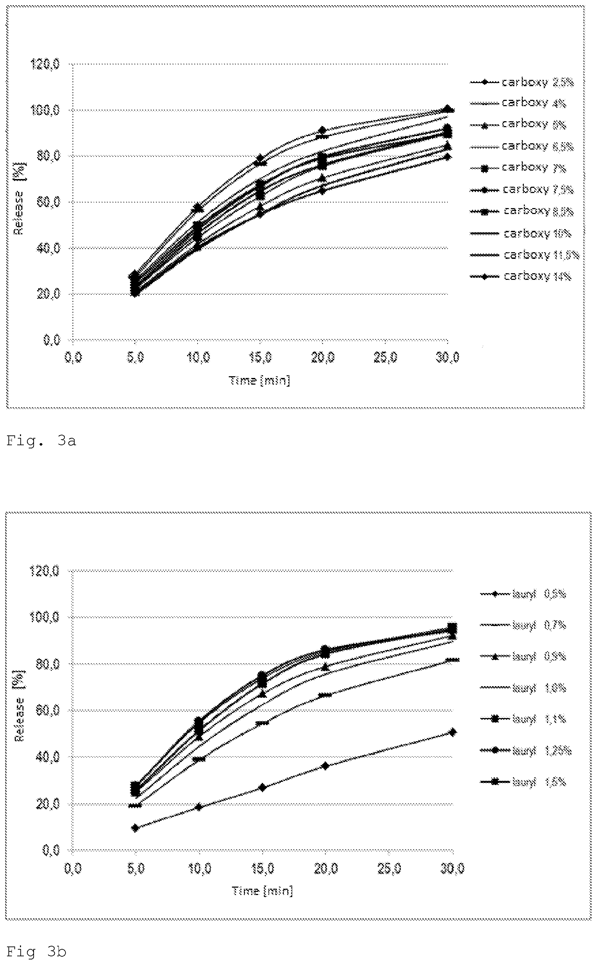

Considering previously obtained results, further developmental studies of a medicinal product containing a combination of two active substances: inosine pranobex (1000 mg) and zinc ions (6.25 mg) in the form of zinc gluconate, in the form of a tablet was conducted towards the development of the product formulation, choosing the appropriate ratio of disintegrating agents and increasing the rate of API dissolution. The following are formulation tests performed by selecting the sodium starch glycolate (type A) excipient concentrations from 2.5% to 14% and the following sodium lauryl sulfate excipient concentrations: 0.5% to 1.5%. The results obtained are summarized in Tables 5a and 5b and in Figures 3a and 3b where Figure 3a depicts the release profiles of inosine pranobex in tests using different quantitative ratios of a disintegrating agent (sodium carboxymethyl starch) in phosphate buffer pH = 4.5 and Figure 3b shows release profiles of inosine pranobex in tests using different quantitative ratios of the excipient increasing the dissolution rate of active substances (sodium lauryl sulfate) in phosphate buffer pH = 4.5.

As a result of the conducted tests, it was found that the consistency of the release profile of the active substance pranobex inosine from a medicinal product containing a combination of two active substances: inosine pranobex (1000 mg) and zinc ions (6.25 mg) in the form of zinc gluconate, with the release profile of API from the reference product, was obtained for product composition with a percentage of sodium carboxymethyl starch (type A) in the range of 5 - 14% and sodium lauryl sulfate at a level of 0.7 to 1%. Despite the positive test results for the formulation with 14% of sodium starch glycolate, further increase of its level in the product is unreasonable, because this value already at this level exceeds the recommended amount according to the literature data of the excipient, while maintaining its disintegrating function in the solid form of the drug.

For the above technological tests, the final release of zinc ions was determined. An acceptable level of zinc ion release at a level exceeding 80% of its value in the formulation during 45 min was observed for all tests with the levels of substances accelerating the release: sodium starch glycolate (type A) in the range from 5% to 14% and from 0.7 % to 1% of sodium lauryl sulfate. Due to the fact that for this level the assumed consistency of the inosine pranobex release profiles and the appropriate level of zinc ion release have been surprisingly found, the determined range of release modifying substances is considered appropriate. Surprisingly, it turned out that the final release of the second active substance used (zinc ions in the form of zinc gluconate) in the developed formulation of the medicinal product Inosine pranobex + Zinc gluconate, 1000 mg + 6.25 mg tablet, meets the tightened criteria of 80% release in 20 minutes only for one of the formulations tested, which contains 7% sodium carboxymethyl starch (type A) in combination with 1% sodium lauryl sulfate, according to the essence of the invention.

### Example 4. Determination an appropriate amount of filling agent (mannitol).

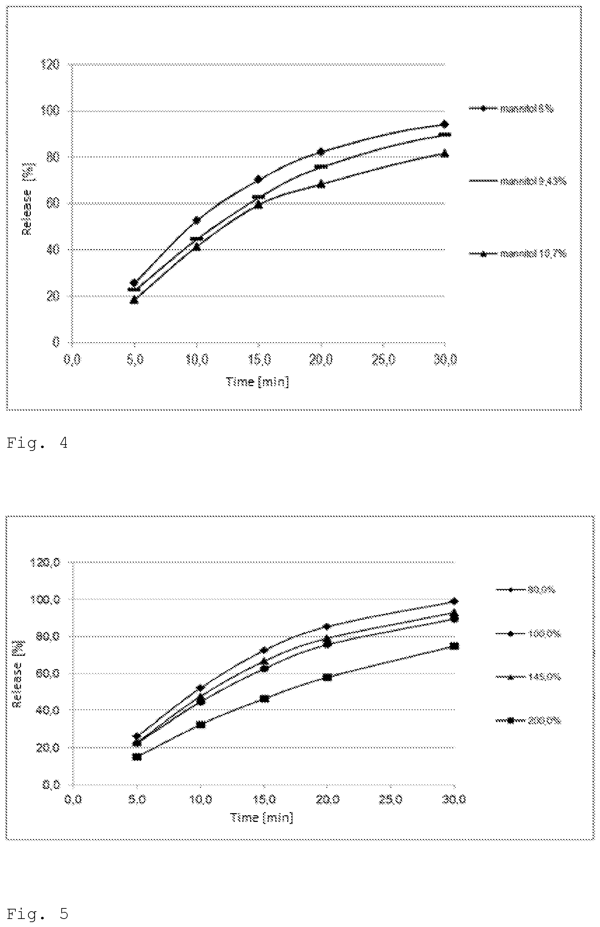

In order to select the correct amount of filling agent: mannitol, technological tests were carried out using different percentage concentration of this substance in the quantitative composition of the invention. The composition of the tests together with the results obtained are presented in the following Table 6 and Figure 4, which illustrate the release profiles of inosine pranobex from Inosine pranobex + Zinc gluconate 1000 mg + 6.25 mg tablets, tablets for tests with different percentages of mannitol, in phosphate buffer pH = 4.5.

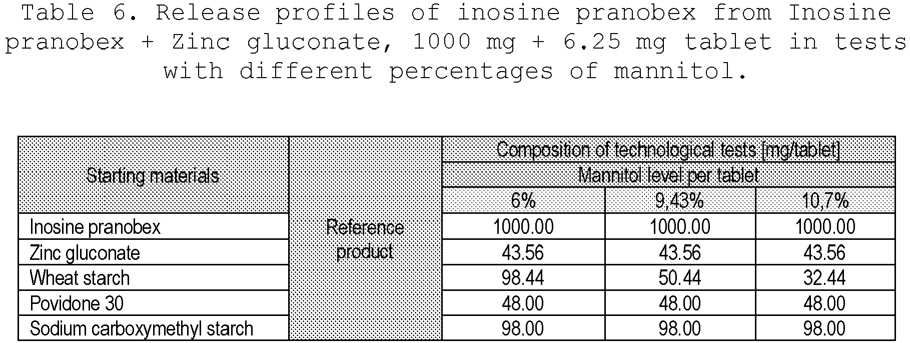

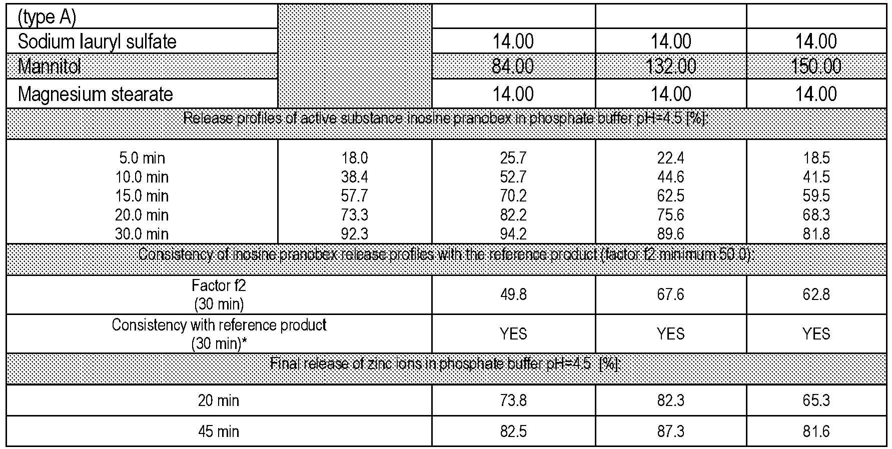

The content of mannitol as a filling excipient at the level of 6% determines the product whose release profile of inosine pranobex was at the acceptability limits, and the amount of zinc ions released meets the assumptions of at least 80% within 45 min. Therefore, the amount of mannitol for the first test is considered to be the limit amount below which the release profile will not meet the assumptions made. The use of the examined excipient at the level of 9.43% and 10.7% determines the preparation of tablets whose release profiles of inosine pranobex relative to the reference product are at an acceptable level (similarity factor f2 above 50 compared to the reference product) and the amount of zinc ions released meets the assumptions (at least 80% during 45 min). Surprisingly, it turned out that only at a level of mannitol consistent with the disclosed composition, the level of zinc ion release exceeds 80% already in 20 min.

Taking into account the release profile of inosine pranobex as well as the final release of zinc ions, it was found that the content of the filling agent mannitol at a level from 6% to 10.7% of the tableting mixture determines the preparation of tablets that meet all assumed quality requirements for the medicinal product.

### Example 5. Selection of the amount of active substance: zinc gluconate.

The next stage of developmental research involved determining the amount of zinc gluconate (source of zinc ions) to be used in Inosine pranobex + Zinc gluconate, 1000 mg + 6.25 mg tablet. It was assumed that zinc ions must be released from the tested drug form in an amount of at least 80% of their initial content in the product within 45 minutes. However, the release profile of the other active substance inosine pranobex must be consistent with the market reference product. Technological tests were carried out in accordance with the recipe described in Example 1, however, the composition was modified using different contents of zinc gluconate (from 80% to 200% of its initial amount in the tested product). The results obtained for the performed tests of release of active substances Inosine pranobex + Zinc gluconate, 1000 mg + 6.25 mg tablet, are presented in Table 7 and Figure 5, which shows the release profiles of inosine pranobex from Inosine pranobex + Zinc gluconate 1000 mg + 6.25 mg tablets, in tests using different percentages of zinc gluconate in phosphate buffer pH = 4.5.

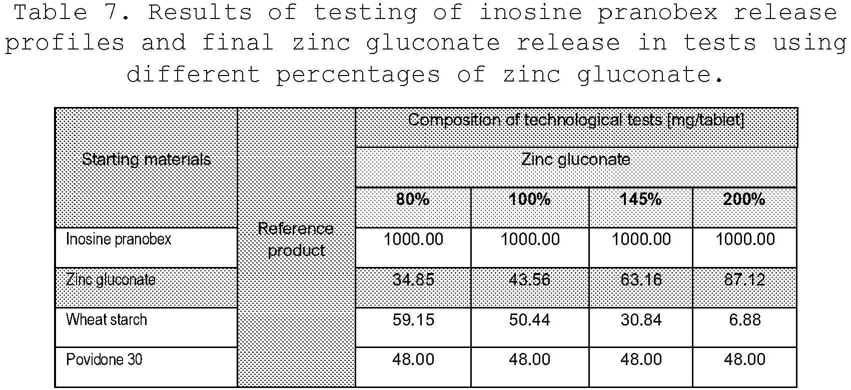

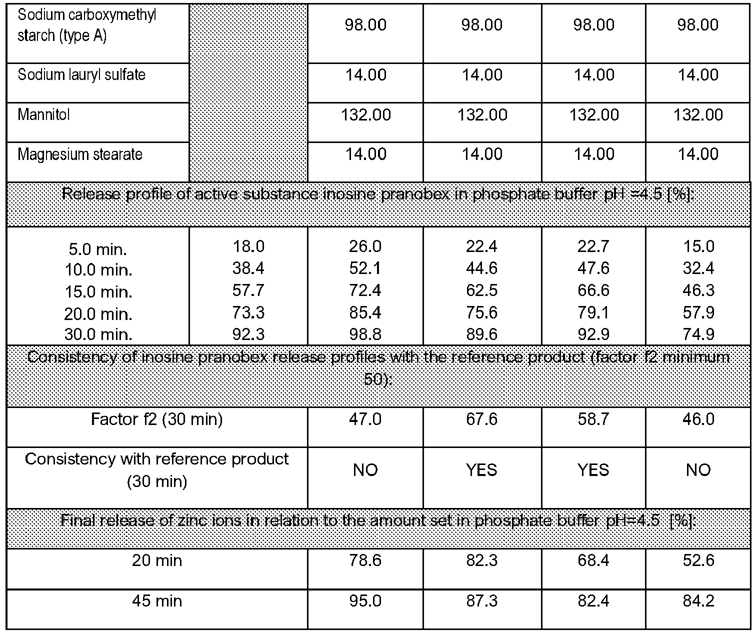

As a result of the conducted research, it was found that the consistency of the release profile of the active substance pranobex inosine from a medicinal product containing a combination of two active substances: inosine pranobex (1000 mg) and zinc ions (6.25 mg) in the form of zinc gluconate in the form of a tablet, with the release profile of API from comparative product, was obtained for product compositions with a percentage of zinc gluconate in the range from 100% to 145%. The final release of zinc ions for the above zinc gluconate range met the acceptability criteria. Based on the tests carried out, it was surprisingly found that only for the composition disclosed in example 1, the final amount of zinc ion release exceeded 80% in just 20 minutes. The range of zinc gluconate content determined during the tests in the present invention was determined to be in the range from 100% to 145% in relation to the composition presented in Example 1.

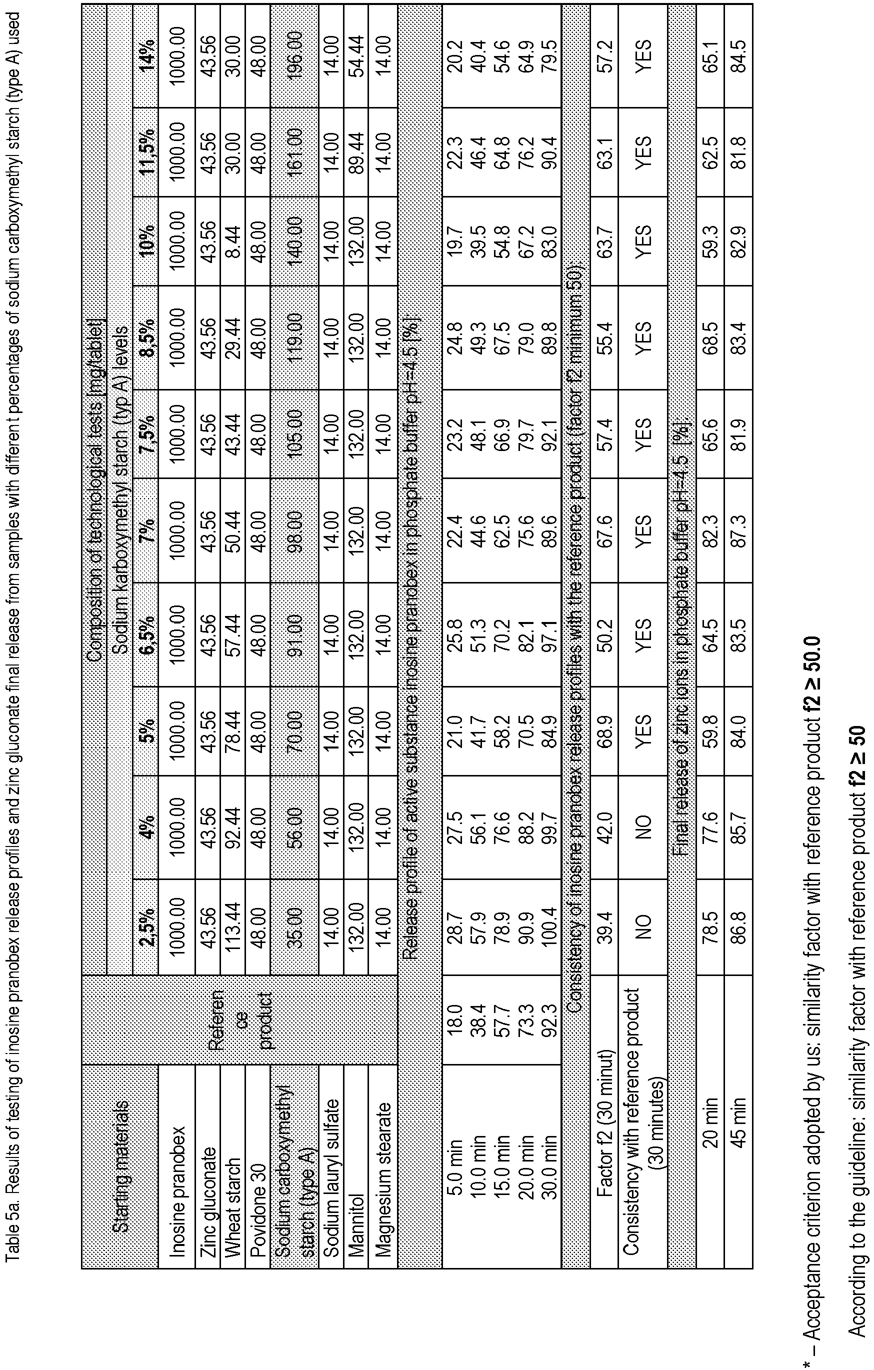

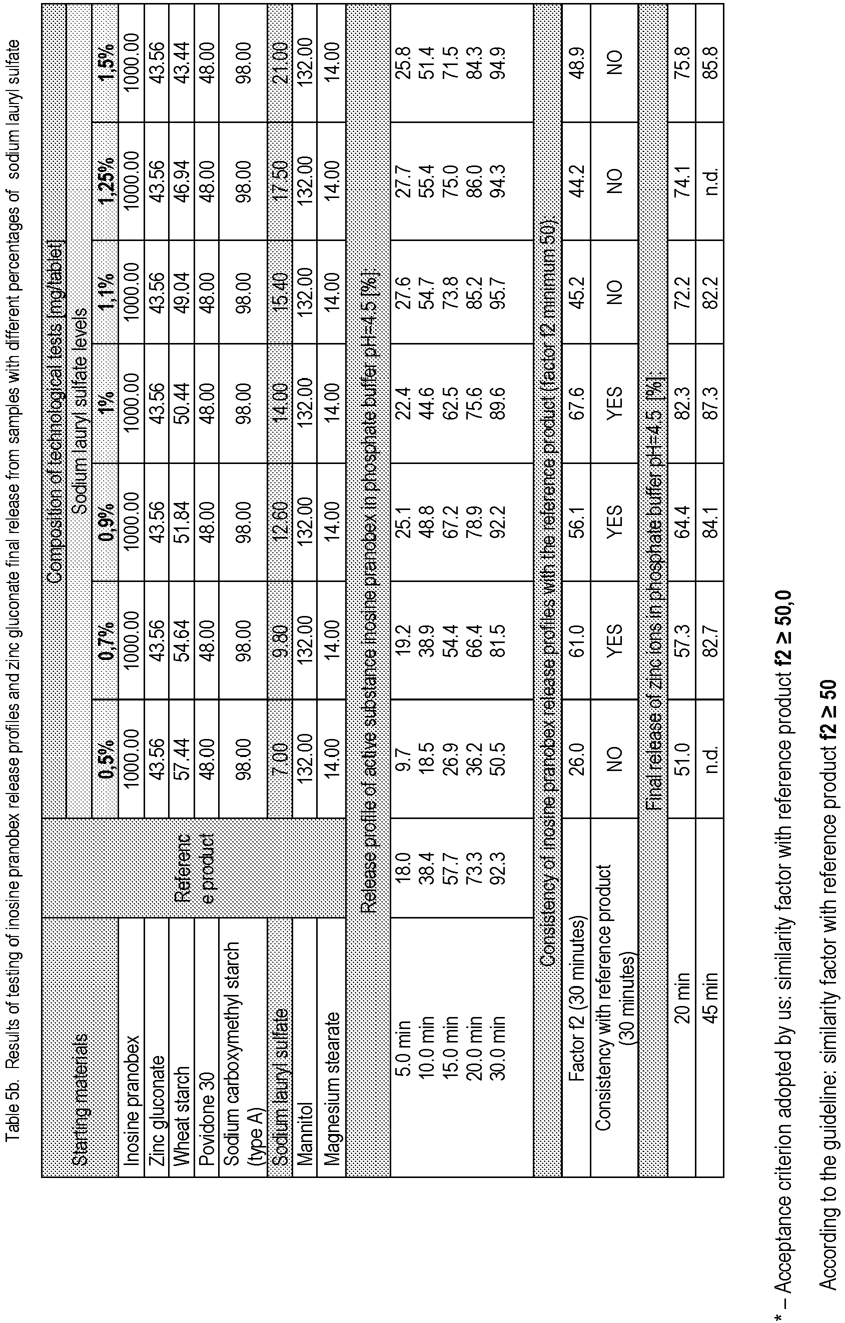

Example 6. Use of a solid dosage form containing zinc gluconate and inosine proanobex.

Inosine pranobex and zinc ions modulate similar immune response processes. Both zinc and inosine pranobex affect the activity of NK cells, macrophages and neutrophils. They also modulate the process of phagocytosis and affect the chemotaxis of cells of the immune system. In addition, zinc regulates the adhesion of neutrophils to vascular endothelial cells. Both substances regulate the secretion of proinflammatory cytokines. Inosine pranobex and zinc modulate the activity and processes of multiplication and differentiation of T lymphocytes. Zinc deficiency leads to a disturbance in the quantity and quantitative ratio of individual types of T lymphocytes. Zinc ions regulate TNFα secretion, whereas inosine pranobex positively affects INFγ secretion. Furthermore, inosine pranobex and zinc regulate the cytotoxicity of T lymphocytes. In addition, inosine pranobex increases the level of IgG immunoglobulin, and zinc is necessary for the reaction of the receptor on the NK surface with the MHC-1 complex on target cells. The decrease in zinc content in the course of infection can be caused by the use of its resources in the body, both by the cells of its own immune system, and by a pathogen using it for its own metabolic needs. Insufficient zinc content in the immune system can disrupt the aforementioned processes associated with the immune response. The immune system is particularly sensitive to fluctuations in zinc levels. Its cells react to a decrease in the level of zinc faster than it is seen in the concentration of this element in plasma. Therefore, a deficiency of this element may lead to an increase in the duration of the disease and hinder treatment, including antiviral therapy.

The effect of inosine pranobex, in the event of zinc deficiency, may be reduced. Adequate zinc concentration determines the correct immune response, while inosine pranobex may additionally strengthen it. It is therefore justified to supplement antiviral and immunomodulating therapy with this compound, a highly bioavailable zinc salt, which is gluconate.

The described pharmaceutical composition is administered to a human in amounts that ensure the intake of 500 mg to 1000 mg of inosine pranobex in one solid drug form and from 21.78 mg to 63.16 mg of zinc gluconate in one solid drug form.

The solid dosage form containing zinc gluconate and inosine pranobex is used in modulating the immune response and antiviral activity at the recommended dose of 50 mg per kg of body weight per day of inosine pranobex dosed with 1 tablet containing 1000 mg of inosine pranobex and 6.25 mg of zinc ions or tablets containing 500 mg inosine pranobex and 3.125 mg zinc ions, 3 to 4 times a day in the adult population and half a tablet containing 1000 mg inosine pranobex and 6.25 mg zinc ions, or one or half of tablet containing 500 mg inosine pranobex and 3.125 mg of zinc ions 3 to 4 times a day in the pediatric population.

## Claims

1. A solid dosage form containing zinc gluconate and inosine pranobex and adjuvants, **characterized in that** zinc gluconate is in an amount of 3.11% to 4.51% by mass, inosine pranobex is in amount of 71.43% by mass, sodium carboxymethyl starch (type A) is in the range from 5% to 14% by weight of the dosage form and sodium lauryl sulfate is at a level from 0.7% to 1% by weight of the dosage form and the mannitol content is from 6% to 10.7% by weight of the dosage form.

2. The dosage form according to claim 1, **characterized in that** it contains magnesium stearate, preferably in an amount of 1%, povidone, preferably in an amount of 3.43%, and starch, preferably wheat starch in an amount of 3.6%.

3. A method of obtaining a solid dosage form as defined in the first claim, **characterized in that** it comprises
a) forming a gruel for the wet granulation process, preferably in an amount of 6.06% by weight of the dosage form based on water, povidone 30 and sodium lauryl sulfate, preferably at povidone temperature from 30 °C to 60 °C, lauryl sulfate temperature from 30 °C to 35 °C, for 3.5 hours
b) pre-mixing of inosine pranobex with zinc gluconate, wheat starch and sodium carboxymethyl starch (type A), preferably at a temperature of 20 °C to 30 °C and with granulator rotating at 100 to 300 rpm
c) spraying the gruel based binder from step a), preferably in the amount determined by the following parameters: pump rotation from 125 rpm to 250 rpm and addition of the binder to the substances from step b) for 2.10 min to 4.40 min, to make granulate
d) drying the granulate from step c), preferably by means of a fluid bed dryer at from 40°C to 50°C
e) mixing the granulate from step d) with mannitol in dry form, preferably sieved through a 1.5 mm to 2.5 mm mesh
f) mixing the granulate from step e) with a lubricant, preferably magnesium stearate, preferably sieved through a 1.5 mm to 2.5 mm mesh
g) tableting, preferably at 10-50 kN pressure forces

4. The solid dosage form defined in claim 1 for use in modulating immune response and antiviral activity by administration to a human.

5. The solid dosage form according to claim 4 for use in modulating immune response and antiviral activity, J Z **characterized in that** the solid dosage form is administered to a human in amounts that ensure the intake of 500 mg to 1000 mg of inosine pranobex in one solid dosage form, and 21.78 mg to 63.16 mg of zinc gluconate in one solid dosage form.

6. The solid dosage form according to claims 4 and 5 for use in modulating immune response and antiviral activity, J **characterized in that** the recommended dose is 50 mg per kg of body weight per day of inosine pranobex administered with 1 tablet containing 1000 mg inosine pranobex and 6.25 mg zinc ions or 2 tablets containing 500 mg inosine pranobex and 3.125 mg zinc ions 3 to 4 times per day in the adult population and a half tablet containing 1000 mg inosine pranobex and 6.25 mg zinc ions or 1 or a half of tablet containing 500 mg inosine pranobex and 3.125 mg zinc ions, 3 to 4 times per day in the population pediatric.

## Patentansprüche

1. Feste Darreichungsform, enthaltend Zinkgluconat und Inosinpranobex und Hilfsstoffe, **dadurch gekennzeichnet, dass** Zinkgluconat in einer Menge von 3,11 bis 4,51 Masse-%, Inosinpranobex in einer Menge von 71,43 Gew.-%, Natriumcarboxymethylstärke (Typ A) im Bereich von 5 bis 14 Gew.-% der Darreichungsform liegt und Natriumlaurylsulfat in einer Menge von 0,7 bis 1 Gew.-% der Darreichungsform vorliegt und der Mannitolgehalt 6 bis 10,7 Gew.-% der Darreichungsform beträgt.

2. Darreichungsform nach Anspruch 1, **dadurch gekennzeichnet, dass** sie Magnesiumstearat, bevorzugt in einer Menge von 1%, Povidon, bevorzugt in einer Menge von 3,43%, und Stärke, bevorzugt Weizenstärke, in einer Menge von 3,6% enthält.

3. Verfahren zum Erlangen einer festen Darreichungsform, wie sie im ersten Gegenstand der Erfindung definiert ist, **dadurch gekennzeichnet, dass** es umfasst
a) Bilden eines Breis für das Nassgranulationsverfahren, bevorzugt in einer Menge von 6,06 Gew.-% der Darreichungsform, bezogen auf Wasser, Povidon 30 und Natriumlaurylsulfat, bevorzugt bei einer Povidontemperatur von 30°C bis 60°C, Laurylsulfattemperatur von 30°C bis 35°C, für 3,5 Stunden
b) Vormischen von Inosinpranobex mit Zinkgluconat, Weizenstärke und Natriumcarboxymethylstärke (Typ A), bevorzugt bei einer Temperatur von 20°C bis 30°C und mit einem Granulator, der sich mit 100 bis 300 U/min dreht,
c) Versprühen des auf Haferschleim basierenden Bindemittels aus Schritt a), bevorzugt in der Menge, die durch die folgenden Parameter bestimmt wird: Pumpendrehung von 125 U/min bis 250 U/min und Zugabe des Bindemittels zu den Substanzen aus Schritt b) für 2,10 min bis 4,40 min, um Granulat herzustellen,
d) Trocknen des Granulats aus Schritt c), bevorzugt mittels eines Wirbelschichttrockners bei 40°C bis 50°C,
e) Mischen des Granulats aus Schritt d) mit Mannit in trockener Form, bevorzugt gesiebt vermittels 1,5 mm bis 2,5 mm Mesh,
f) Mischen des Granulats aus Schritt e) mit einem Schmiermittel, bevorzugt Magnesiumstearat, bevorzugt gesiebt vermittels 1,5 mm bis 2,5 mm Mesh,
g) Tablettierung, bevorzugt bei Druckkräften von 10 - 50 kN.

4. Die in Anspruch 1 definierte feste Darreichungsform zur Verwendung bei der Modulation der Immunantwort und der antiviralen Aktivität durch Verabreichung an einem Menschen.

5. Die feste Darreichungsform nach Anspruch 4 zur Verwendung bei der Modulation der Immunantwort und der antiviralen Aktivität, **dadurch gekennzeichnet, dass** die Darreichungsform einem Menschen in einer Menge verabreicht wird, die die Aufnahme von 500 mg bis 1000 mg Inosin-Pranobex in einer festen Darreichungsform und 21,78 mg bis 63,16 mg Zinkgluconat in einer festen Darreichungsform gewährleistet.

6. Die feste Darreichungsform nach den Ansprüchen 4 und 5 zur Verwendung bei der Modulation der Immunantwort und der antiviralen Aktivität, **dadurch gekennzeichnet, dass** die empfohlene Dosis 50 mg Inosin-Pranobex pro kg Körpergewicht und Tag beträgt, verabreicht mit 1 Tablette, die 1000 mg Inosin-Pranobex und 6,25 mg Zinkionen enthält, oder 2 Tabletten, die 500 mg Inosin-Pranobex und 3,125 mg Zinkionen 3 bis 4 Mal pro Tag bei Erwachsenen und eine halbe Tablette mit 1000 mg Inosin-Pranobex und 6,25 mg Zinkionen oder 1 oder eine halbe Tablette mit 500 mg Inosin-Pranobex und 3,125 mg Zinkionen, 3 bis 4 Mal pro Tag bei Kindern.

## Revendications

1. Forme pharmaceutique solide contenant du gluconate de zinc et de l'inosine pranobex et des adjuvants, **caractérisée en ce que** le gluconate de zinc est compris en une quantité de 3,11 % à 4,51 % en masse, l'inosine pranobex est comprise en une quantité de 71,43 % en masse, de l'amidon sodique de carboxyméthyle (type A) est compris dans la plage de 5 % à 14 % par rapport au poids de la forme pharmaceutique et du sulfate sodique de lauryle est à un niveau allant de 0,7 % à 1 % par rapport au poids de la forme pharmaceutique et la teneur en mannitol est comprise entre 6 % et 10,7 % par rapport au poids de la forme pharmaceutique.

2. Forme pharmaceutique selon la revendication 1, **caractérisée en ce qu'**elle contient du stéarate de magnésium, de préférence en une quantité de 1 %, de la povidone, de préférence en une quantité de 3,43 %, et de l'amidon, de préférence de l'amidon de blé en une quantité de 3,6 %.

3. Procédé d'obtention d'une forme pharmaceutique solide selon la revendication 1, **caractérisé en ce qu'**il comprend les étapes consistant à
a) former un gruau pour le processus de granulation humide, de préférence en une quantité de 6,06 % par rapport au poids de la forme pharmaceutique à base d'eau, de povidone 30 et de sulfate sodique de lauryle, de préférence à une température de povidone allant de 30 °C à 60 °C, à une température de sulfate de lauryle allant de 30 °C à 35 °C, pendant 3,5 heures
b) prémélanger l'inosine pranobex avec le gluconate de zinc, l'amidon de blé et l'amidon sodique de carboxyméthyle (type A), de préférence à une température de 20 °C à 30 °C et avec un granulateur tournant de 100 à 300 tr/min.
c) pulvériser le liant à base de gruau obtenu à l'étape a), de préférence selon la quantité déterminée par les paramètres suivants : une rotation de pompe de 125 tr/min. à 250 tr/min. et une addition du liant aux substances obtenues à l'étape b) pendant 2,10 min. à 4,40 min., pour former un granulat
d) sécher le granulat obtenu à l'étape c), de préférence au moyen d'un séchoir à lit fluidisé à une température allant de 40 °C à 50 °C
e) mélanger le granulat obtenu à l'étape d) avec du mannitol sous forme sèche, de préférence tamisé à travers une maille de 1,5 mm à 2,5 mm
f) mélanger le granulat obtenu à l'étape e) avec un lubrifiant, de préférence du stéarate de magnésium, de préférence tamisé à travers une maille de 1,5 mm à 2,5 mm
g) fabriquer des comprimés de préférence à des forces de pression de 10 à 50 kN.

4. Forme pharmaceutique solide selon la revendication 1, pour utilisation pour la modulation de réponse immunitaire et d'activité antivirale par une administration à un être humain.

5. Forme pharmaceutique solide selon la revendication 4, pour utilisation pour la modulation de réponse immunitaire et d'activité antivirale,
**caractérisée en ce que** la forme pharmaceutique solide est administrée à un être humain selon des quantités qui garantissent la prise de 500 mg à 1000 mg d'inosine pranobex en une seule forme pharmaceutique solide, et de 21,78 mg à 63,16 mg de gluconate de zinc en une seule forme pharmaceutique solide.

6. Forme pharmaceutique solide selon les revendications 4 et 5, pour utilisation pour la modulation de réponse immunitaire et d'activité antivirale,
**caractérisée en ce que** la dose recommandée est de 50 mg par kg de poids corporel par jour d'inosine pranobex administrée par 1 comprimé contenant 1000 mg d'inosine pranobex et 6,25 mg d'ions zinc ou par 2 comprimés contenant 500 mg d'inosine pranobex et 3,125 mg d'ions zinc 3 à 4 fois par jour pour la population adulte, et un demi-comprimé contenant 1000 mg d'inosine pranobex et 6,25 mg d'ions zinc, ou 1 comprimé ou un demi-comprimé contenant 500 mg d'inosine pranobex et 3,125 mg d'ions zinc, 3 à 4 fois par jour pour la population pédiatrique.
